# EUROPEAN PATENT SPECIFICATION

(11) **EP 4 438 669 B1**
(45) Date of publication and mention of the grant of the patent: **13.08.2025**
(21) Application number: 24157881.4
(22) Date of filing: 15.02.2024
(51) Int. Cl.: C08L 1/02, C08K 5/09, C08L 5/08

(54) **CELLULOSE PARTICLE**
CELLULOSEPARTIKEL
PARTICULE DE CELLULOSE

(30) Priority: 28.03.2023 JP 2023052435; 16.11.2023 JP 2023195177
(43) Date of publication of application: 02.10.2024
(73) Proprietor: FUJIFILM Business Innovation Corp., Minato-ku Tokyo (JP)
(72) Inventor: MATOBA, Shota, Minamiashigara-shi, Kanagawa (JP); OKI, Masahiro, Minamiashigara-shi, Kanagawa (JP); ISHIZUKA, Takahiro, Minamiashigara-shi, Kanagawa (JP); YAO, Kenji, Minamiashigara-shi, Kanagawa (JP); TAGUCHI, Tetsuya, Minamiashigara-shi, Kanagawa (JP); HAMANO, Hirokazu, Minamiashigara-shi, Kanagawa (JP)
(74) Representative: Kurig, Thomas

(56) References cited:
- FREEMAN A ET AL: "Site-protected fixation and immobilization of Escherichia coli cells displaying surface-anchored beta-lactamase", BIOTECHNOLOGY AND BIOENGINEERING, JOHN WILEY, HOBOKEN, USA, vol. 62, no. 2, 20 January 1999 (1999-01-20), pages 155 - 159, XP002374060, ISSN: 0006-3592, DOI: 10.1002/(SICI)1097-0290(19990120)62:2<155::AID-BIT4>3.0.CO;2-U
- WEI LIQING ET AL: "Chemical modification of nanocellulose with canola oil fatty acid methyl ester", CARBOHYDRATE POLYMERS, vol. 169, 1 August 2017 (2017-08-01), GB, pages 108 - 116, XP093182445, ISSN: 0144-8617, DOI: 10.1016/j.carbpol.2017.04.008

## Description

### BACKGROUND OF THE INVENTION

### (i) Field of the Invention

The present invention relates to a cellulose particle.

### (ii) Description of Related Art

JP7161073B discloses "a water-repellent cellulose bead which is obtained by subjecting a cellulose bead to a surface treatment with hydrogenated lecithin having a phosphatidylcholine content of 40% to 85% by mass and in which the amount of the hydrogenated lecithin with respect to 100 parts by mass of the cellulose bead is 0.1 to 5 parts by mass".

JP2003-146829A discloses "a microcrystalline cellulose powder that is characterized by being subjected to a surface treatment with metallic soap or hydrogenated lecithin".

JP2022-131884A discloses "a surface-treated cellulose powder obtained by coating a surface of a cellulose powder with a salt between each of silicic acid, alginic acid, fatty acid, and lysine dilauroyl glutamate, and one or more metals selected from calcium, aluminum, and magnesium".

JP2019-510032A discloses "a cosmetic powder including at least one kind of cosmetic powder, where a surface of the at least one kind of cosmetic powder is chemically modified by at least one polysaccharide, a salt form thereof, or a mixture thereof, and the at least one polysaccharide is chemically immobilized to the surface of the at least one kind of powder".

### SUMMARY OF THE INVENTION

An object of the present invention is to provide a cellulose particle excellent in water repellency and moist feeling even in a case of being exposed to high temperature water for a long period of time, as compared with a cellulose particle having a mother particle containing cellulose as a main component, where the cellulose particle has, in the following order, a first coating layer that contains a cellulose particle having a single coating layer on the surface of the mother particle or contains a polysaccharide having no primary amine or secondary amine on the surface of the mother particle, and a second coating layer containing calcium stearate.

There is provided a cellulose particle excellent in water repellency and moist feeling even in a case of being exposed to high temperature water for a long period of time, as compared with a cellulose particle having a mother particle containing cellulose as a main component, where the cellulose particle has, in the following order, a first coating layer that contains a cellulose particle having a single coating layer on the surface of the mother particle or contains a polysaccharide having no primary amine or secondary amine on the surface of the mother particle, and a second coating layer containing calcium stearate.

There is provided a cellulose particle excellent in water repellency and moist feeling even in a case of being exposed to high temperature water for a long period of time, as compared with a case where the polysaccharide is a lactic acid salt of chitosan.

There is provided a cellulose particle excellent in water repellency and moist feeling even in a case of being exposed to high temperature water for a long period of time, as compared with a case where the hydrophobic compound is at least one selected from a fatty acid or an acylated amino acid.

There is provided a cellulose particle excellent in water repellency and moist feeling even in a case of being exposed to high temperature water for a long period of time, as compared with a case where the hydrophobic compound is an acylated amino acid salt.

There is provided a cellulose particle excellent in water repellency and moist feeling even in a case of being exposed to high temperature water for a long period of time, as compared with a case where the fatty acid metal salt is magnesium stearate.

There is provided a cellulose particle excellent in water repellency and moist feeling even in a case of being exposed to high temperature water for a long period of time, as compared with a case where the acylated amino acid salt is calcium myristoyl glutamate.

There is provided a cellulose particle excellent in water repellency and moist feeling even in a case of being exposed to high temperature water for a long period of time, as compared with a case where the polysaccharide is a lactic acid salt of chitosan or the hydrophobic compound is magnesium stearate.

There is provided a cellulose particle in which the coating amount of the first coating layer with respect to the mother particle may be less than 0.2% by mass or more than 10% by mass.

There is provided a cellulose particle excellent in water repellency and moist feeling even in a case of being exposed to high temperature water for a long period of time, as compared with a case where the coating amount of the first coating layer with respect to the mother particle is less than 0.25% by mass or more than 3% by mass.

There is provided a cellulose particle excellent in water repellency and moist feeling even in a case of being exposed to high temperature water for a long period of time, as compared with a case where the coating amount of the second coating layer with respect to the mother particle is less than 0.5% by mass or more than 25% by mass.

There is provided a cellulose particle excellent in water repellency and moist feeling even in a case of being exposed to high temperature water for a long period of time, as compared with a case where the coating amount of the second coating layer with respect to the mother particle is less than 3% by mass or more than 12% by mass.

There is provided a cellulose particle excellent in water repellency and moist feeling even in a case of being exposed to high temperature water for a long period of time, as compared with a case where the ratio of the first coating amount to the coating amount of the second coating layer is less than 0.04 or more than 2.

There is provided a cellulose particle excellent in water repellency and moist feeling even in a case of being exposed to high temperature water for a long period of time, as compared with a case where the volume average particle diameter of the cellulose particles is less than 3 µm or more than 10 µm.

### DETAILED DESCRIPTION OF THE INVENTION

Hereinafter, an exemplary embodiment of the present invention will be described in detail.

Regarding ranges of numerical values described stepwise in the present specification, the upper limit value or lower limit value of a range of numerical values may be replaced with the upper limit value or lower limit value of another range of numerical values described stepwise. In addition, in a range of numerical values described in the present specification, the upper limit value or lower limit value of the range of numerical values may be replaced with a value shown in Examples.

In the present specification, the term "step" is included in the present term as long as an intended purpose of the step is achieved not only as an independent step but also in a case where the step is not clearly distinguished from other steps.

Each component may include a plurality of corresponding substances.

In a case where the amount of each component is mentioned and in a case where a plurality of kinds of substances corresponding to each component are present, the amount means a total amount of the plurality of kinds of substances unless otherwise specified.

"(Meth)acrylic" means at least one of acrylic or methacryl, and "(meth)acrylate" means at least one of acrylate or methacrylate.

### <Cellulose Particle>

A cellulose particle according to the present exemplary embodiment has;
a mother particle containing cellulose as a main component (hereinafter, also referred to as a "cellulose mother particle"),
a first coating layer containing at least one polysaccharide selected from the group consisting of a basic polysaccharide and a salt of the basic polysaccharide, the first coating layer being provided on a surface of the mother particle, and
a second coating layer containing at least one hydrophobic compound selected from a fatty acid, a fatty acid metal salt, an acylated amino acid, or an acylated amino acid salt, the second coating layer being provided on the first coating layer.

With the above configuration, the cellulose particle according to the present exemplary embodiment is excellent in water repellency and moist feeling even in a case of being exposed to high temperature water for a long period of time. That is, the cellulose particle according to the present exemplary embodiment is excellent in the water repellency and the maintainability of the moist feeling with respect to high-temperature warm water. The reason for this effect is presumed as follows.

Due to having high biodegradability and toughness, cellulose particles are expected as a substitute for resin particles in various fields including use applications in cosmetics. In addition, in association with the increasing functionality of products for each use application, cellulose particles are required to have various functions.

For one of the various functions, a hydrophobic treatment is effective as a means for imparting a function. For example, the hydrophobic treatment can impart water repellency and a moist feeling to cellulose particles. For example, in a case where cellulose particles having water repellency and a moist feeling are applied to use applications in cosmetics, hydrophobic treatment is possible to suppress smearing of makeup due to sweat and to impart a smooth skin feeling.

For example, there is a technique in which the surface of the cellulose mother particle is subjected to a hydrophobic treatment with a hydrophobic compound such as hydrogenated lecithin or metallic soap, thereby imparting water repellency and a smooth skin feeling (for example, JP7161073B, JP2003-146829A, JP2022-131884A, or the like). However, there is no chemical interaction between the cellulose mother particle and the coating layer due to the hydrophobic compound. Therefore, the binding force between the cellulose mother particle and the coating layer is weak, and a part of the surface of the cellulose mother particle is not coated in a nearly uniform state and is exposed. As a result, water molecules invade the inside of the cellulose mother particles in which the intermolecular hydrogen bonds are loosened, for example, in a case where the cellulose particles are exposed to the manufacturing step or the high temperature water at a temperature higher than 50°C. As a result, the coating layer may be easily peeled off due to the swelling or the like of the cellulose mother particles. That is, water repellency and maintenance of a favorable smooth skin feeling are deficient.

In addition, for example, in a case where a coating layer made from a hydrophilic polysaccharide is formed on a surface of a cellulose mother particle, there is a technique for immobilizing the polysaccharide that becomes a coating layer, by interposing an intermediate layer made from an aggregating agent between the mother particle and the coating layer (for example, JP2019-510032A or the like). Due to the anchoring effect of the aggregating agent, the intermediate layer enhances the stability with respect to heat. Therefore, it is expected that a moist feeling derived from the hydrophilic polysaccharide constituting the coating layer can be easily obtained.

However, although the dispersibility of the cellulose particles in water is improved since the hydrophilic polysaccharide is hydrophilic, it is difficult to maintain the moist feeling due to the dissolution or the like of the hydrophilic polysaccharide in water in a case where the cellulose particles are exposed to high temperature water. In addition, water repellency cannot be imparted to the cellulose particles.

On the other hand, the cellulose particle according to the present exemplary embodiment adopts a configuration in which a first coating layer containing at least one polysaccharide selected from the group consisting of a basic polysaccharide and a salt of the basic polysaccharide, and a second coating layer containing at least one hydrophobic compound selected from a fatty acid, a fatty acid metal salt, an acylated amino acid, or an acylated amino acid salt are sequentially formed on the surface of the cellulose mother particle.

The basic polysaccharide and a salt thereof, which constitute the first coating layer interposed between the cellulose mother particles and the second coating layer which is the outermost surface layer, have a structure having a large number of hydroxyl groups, which is similar to the structure of cellulose. Therefore, the basic polysaccharide and a salt thereof form a nearly uniform layer while strongly interacting with cellulose by an intermolecular hydrogen bond.

In addition, due to having an amino group, the basic polysaccharide and a salt thereof attract and concurrently immobilize a hydrophobic compound having a carbonyl group on the particle surface. In particular, chitosan is known as an aggregating agent, and it is possible to more strongly immobilize cellulose and a hydrophobic compound by an aggregation effect. Due to having a carbonyl group, the fatty acid, the fatty acid metal salt, the acylated amino acid, and the acylated amino acid salt, as the hydrophobic compound, are more firmly immobilized to the basic polysaccharide and a salt thereof. These work on the exhibition and maintenance of the water repellency.

In addition, a layer formed by the basic polysaccharide and a salt thereof is poorly soluble in water but hydrophilic, and thus water molecules are adsorbed, and a moist feeling is exhibited. At the same time, because of the poorly soluble property in water, the water molecules are prevented from invading the inside of the cellulose particles, the details of which are not clear.

Therefore, even in a case of being exposed to high temperature water for a long period of time, the cellulose mother particles are not swelled and the hydrophobic compound is not peeled off, and thus high water repellency and a moist feeling are maintained.

From the above, it is presumed that the cellulose particle according to the present exemplary embodiment is excellent in water repellency and moist feeling even in a case of being exposed to high temperature water for a long period of time.

Hereinafter, the cellulose particle according to the present exemplary embodiment will be described in detail.

### (Mother Particle)

The mother particle is a target particle on which the first coating layer and the second coating layer are formed, and contain cellulose as a main component.

Here, the phrase "containing cellulose as a main component" refers to that the content of cellulose with respect to the mother particle is 90% by mass or more (for example, preferably 95% by mass or more, 98% by mass or more, or 100% by mass).

The number average molecular weight of cellulose is, for example, preferably 37,000 or more and more preferably 45,000 or more.

The upper limit value of the number average molecular weight of cellulose is not particularly limited and may be, for example, 100,000 or less.

In a case of setting the number average molecular weight of cellulose to 37,000 or more, the biodegradability and the moist feeling are enhanced. The reason for this effect is presumed as follows.

In a case where the number average molecular weight is 37,000 or more, the increase of the terminal hydroxyl groups is suppressed, the number of hydroxyl groups per unit volume is reduced, and the number of intramolecular/intermolecular hydrogen bonds and the bond strength thereof are reduced. As a result, the flexibility of the cellulose particle is increased, and the moist feeling is likely to be improved.

The number average molecular weight of cellulose is measured by a gel permeation chromatography method (differential refractive index meter: Optilab T-rEX, manufactured by Wyatt Technology Corporation, multi-angle light scattering detector: DAWN HELEOS II, manufactured by Wyatt Technology Corporation, TSKgel α-M column: one α-3000 column, manufactured by Tosoh Corporation) using dimethylacetamide (added with 0.1 M lithium chloride) as a solvent.

### - Other Components -

The mother particle may contain other components.

Examples of the other components include a plasticizer, a flame retardant, a compatibilizer, a mold release agent, a light fastener, a weather resistant agent, a colorant, a pigment, a modifier, a drip inhibitor, an antistatic agent, a hydrolysis inhibitor, a filler, a reinforcing agent (glass fiber, carbon fiber, talc, clay, mica, glass flake, milled glass, glass beads, crystalline silica, alumina, silicon nitride, aluminum nitride, boron nitride, or the like), an acid acceptor for preventing acetic acid release (an oxide such as magnesium oxide or aluminum oxide; a metal hydroxide such as magnesium hydroxide, calcium hydroxide, aluminum hydroxide, or hydrotalcite; calcium carbonate; talc; or the like), and a reactive trapping agent (for example, an epoxy compound, an acid anhydride compound, or a carbodiimide).

The content of the other components is, for example, preferably 0% by mass or more and 5% by mass or less with respect to the total amount of the mother particles. Here, "0% by mass" means that other components are not contained.

### (First Coating Layer)

The first coating layer is an intermediate layer interposed between the cellulose mother particles and the second coating layer.

The first coating layer contains at least one polysaccharide selected from the group consisting of a basic polysaccharide and a salt of the basic polysaccharide.

The basic polysaccharide is a polysaccharide having a primary amine or a secondary amine as a basic functional group. Examples of the basic functional group contained in the polysaccharide include an amino group and an acetamide group.

Examples of the basic polysaccharide include chitosan, chitin, aminated cellulose, and hyaluronic acid.

Examples of the salt of the basic polysaccharide include a lactic acid salt of the basic polysaccharide, a citric acid salt thereof, a butyric acid salt thereof, a hydrochloric acid salt thereof, and a sodium salt thereof.

Among these, for example, chitosan or a lactic acid salt of chitosan is preferable, and chitosan is more preferable from the viewpoint of improving the water repellency and the maintainability of the moist feeling with respect to high-temperature warm water.

Here, chitosan is a deacetylated product of chitin. From the viewpoint of improving the water repellency and the maintainability of the moist feeling with respect to high-temperature warm water, the degree of deacetylation of chitosan is, for example, preferably 70% to 100% from the viewpoint of improving the moist feeling.

From the viewpoint of improving the water repellency and the maintainability of the moist feeling with respect to high-temperature warm water, the coating amount of the first coating layer on the mother particle is, for example, preferably 0.2% by mass or more and 10% by mass or less, more preferably 0.25% by mass or more and 3% by mass or less, and still more preferably 0.8% by mass or more and 1.5% by mass or less.

Here, the content of the polysaccharide with respect to the entire first coating layer is, for example, preferably 90% by mass or more and 100% by mass or less and more preferably 95% by mass or more and 100% by mass or less.

### (Second Coating Layer)

The second coating layer is a layer that is provided on the first coating layer and constitutes the outermost layer of the cellulose particle.

The second coating layer contains at least one hydrophobic compound selected from a fatty acid, a fatty acid metal salt, an acylated amino acid, or an acylated amino acid salt.

### - Fatty Acid and Fatty Acid Metal Salt -

The fatty acid is a saturated or unsaturated fatty acid which is linear or branched. The fatty acid may be a mixture of a saturated fatty acid and an unsaturated fatty acid.

From the viewpoint of improving the water repellency and the maintainability of the moist feeling with respect to high-temperature warm water, the fatty acid is, for example, a fatty acid having 14 or more and 22 or less carbon atoms (for example, preferably 18 or more and 20 or less carbon atoms). Specific examples of the linear fatty acid having 14 or more and 22 or less carbon atoms include stearic acid, behenic acid, arachidic acid, palmitic acid, isostearic acid, and myristic acid.

Examples of the fatty acid metal salt include the metal salts of the fatty acid, which are exemplified above.

Examples of the metal in the fatty acid metal salt include metals which are divalent or higher valent.

Examples of the metal in the fatty acid metal salt include magnesium, calcium, aluminum, barium, and zinc.

Here, in the fatty acid and the fatty acid metal salt, in a case where the fatty acid has 14 or more carbon atoms, the packing modes between fatty acid groups are sufficiently diversified, and the crystallinity is suppressed. Therefore, the flexibility is increased, and the moist feeling is improved. On the other hand, in a case where the fatty acid has 22 or less carbon atoms, a fatty acid and a fatty acid metal salt, which have a small diameter, are precipitated to coat the surface of the particle in a nearly uniform state, and thus high water repellency is imparted.

### - Acylated Amino Acid and Acylated Amino Acid Salt -

The acylated amino acid is an amino acid having an acyl group.

Examples of the acyl group include a group represented by a formula; -C(=O)R [R represents a hydrogen atom, a saturated aliphatic group, an unsaturated aliphatic group, or an aromatic group].

From the viewpoint of improving the water repellency and the maintainability of the moist feeling with respect to high-temperature warm water, in the formula; -C(=O)R, R is, for example, preferably a saturated aliphatic group having 12 or more and 18 or less carbon atoms, or an unsaturated aliphatic group having 12 or more and 18 or less carbon atoms.

Examples of the acylated amino acid include myristoyl glutamate, lauroyl glutamate, stearoyl glutamate, lauroyl aspartate, lysine dilauroyl glutamate, palmitoyl glutamate, lauroyl lysine, and lauroyl arginine.

Examples of the acylated amino acid salt include the metal salts of the acylated amino acids, which are exemplified above.

Examples of the metal in the acylated amino acid salt include Na, Ca, Al, Mg, Zn, Zr, and Ti.

From the viewpoint of improving the water repellency and the maintainability of the moist feeling with respect to high-temperature warm water, the acylated amino acid salt is, for example, preferably at least one selected from aluminum stearoyl glutamate or aluminum myristoyl glutamate.

Here, the hydrophobic compound is, for example, preferably a fatty acid metal salt and more preferably calcium stearate. Since these compounds have high water repellency and high affinity for basic polysaccharides and salts of basic polysaccharides, the water repellency and the maintainability of the moist feeling with respect to high temperature warm water are likely to be improved.

From the viewpoint of improving the water repellency and the maintainability of the moist feeling with respect to high-temperature warm water, the coating amount of the second coating layer on the mother particle is, for example, preferably 0.5% by mass or more and 25% by mass or less, more preferably 3% by mass or more and 12% by mass or less, and still more preferably 4% by mass or more and 10% by mass or less.

Here, the content of the hydrophobic compounds with respect to the entire second coating layer is, for example, preferably 90% by mass or more and 100% by mass or less and more preferably 95% by mass or more and 100% by mass or less.

Here, from the viewpoint of improving the water repellency and the maintainability of the moist feeling with respect to high-temperature warm water, the ratio of the coating amount of the first coating layer to the coating amount of the second coating layer is, for example, preferably 0.04 or more and 2 or less, more preferably 0.05 or more and 0.6 or less, and still more preferably 0.08 or more and 0.33 or less.

### (External additive)

An external additive may be added to the cellulose particle according to the present exemplary embodiment.

In a case where an external additive is externally added, the secondary aggregation of the particles is suppressed, and thus the original characteristics of the particles are easily exhibited. As a result, the water repellency and the maintainability of the moist feeling with respect to high-temperature warm water are likely to be improved. In addition, a decrease in biodegradability is also suppressed.

Examples of the external additive include a silicon-containing compound particle, a metallic soap particle, a fatty acid ester particle, and a metal oxide particle.

The silicon-containing compound particle refers to a particle containing silicon.

The silicon-containing compound particle may be a particle containing only silicon or may be a particle containing silicon and other elements.

The silicon-containing compound particle is, for example, preferably a silica particle.

The silica particle may be any particle that contains silica, that is, SiO₂ as a main component, and may be crystalline or non-crystalline. In addition, the silica particle may be a particle manufactured by using a silicon compound such as water glass or an alkoxysilane as a raw material or may be a particle obtained by pulverizing quartz.

The metallic soap particle is a particle containing metallic soap as a main component.

Here, the particle containing metallic soap as a main component refers to a particle in which the content of the metallic soap with respect to the particle is 90% by mass or more.

The metallic soap is a fatty acid metal salt in which a fatty acid and a metal are bonded.

Examples of the fatty acid metal salt include a metal salt of a fatty acid having 10 or more and 25 or less carbon atoms (for example, preferably 12 or more and 22 or less). Examples of the metal salt of a fatty acid having 10 or more and 25 or less carbon atoms include a metal salt of stearic acid, a metal salt of palmitic acid, a metal salt of lauric acid, a metal salt of oleic acid, a metal salt of linoleic acid, and a metal salt of ricinoleic acid.

Examples of the metal in the fatty acid metal salt include a divalent metal.

Examples of the metal in the fatty acid metal salt include magnesium, calcium, aluminum, barium, and zinc.

The fatty acid ester particle is a particle containing a fatty acid ester as a main component.

Here, the particle containing a fatty acid ester as a main component refers to a particle in which the content of the fatty acid ester particle with respect to the particle is 90% by mass or more.

Examples of the fatty acid ester include an esterified product of a saturated fatty acid having 10 or more and 25 or less carbon atoms and an alcohol having 10 or more and 25 or less carbon atoms.

Examples of the fatty acid ester include stearyl stearate, stearyl laurate, and stearyl palmitate.

The metal oxide particle is a particle containing a metal oxide as a main component.

Here, the particle containing a metal oxide as a main component refers to a particle in which the content of the metal oxide with respect to the particles is 90% by mass or more.

As the metal oxide, an oxide of a metal other than silicon can be applied.

Examples of the metal oxide include zinc oxide, magnesium oxide, iron oxide, and aluminum oxide.

From the viewpoint of texture (specifically, moist feeling), the volume average particle diameter of the external additive is, for example, preferably 1 nm or more and 100 nm or less, and more preferably 5 nm or more and 30 nm or less.

The volume average particle diameter of the external additive is measured by the same method as the method for the volume average particle diameter of the cellulose particles.

The amount of the external additive externally added is, for example, preferably 0.1% by mass or more and 2% by mass or less with respect to the total mass of the cellulose particles (cellulose particles in a state where the external additive is not externally added).

### (Volume average particle diameter)

The volume average particle diameter of the cellulose particles according to the present exemplary embodiment is, for example, preferably 3 µm or more and 10 µm or less, more preferably 4 µm or more and 9 µm or less, and still more preferably 5 µm or more and 8 µm or less.

In a case of setting the volume average particle diameter of the cellulose particles according to the present exemplary embodiment to 3 µm or more and 10 µm or less, the particle diameter reaches an appropriate magnitude, the flexibility is increased, and the moist feeling is improved. In addition, the specific surface area also reaches an appropriate magnitude, and thus the first coating layer and the second coating layer can be formed in a nearly uniform state at the time of surface treatment, and high water repellency can be imparted.

The volume average particle diameter of the cellulose particles is measured as follows.

A particle diameter is measured by an LS particle diameter distribution measuring apparatus "Beckman Coulter LS13 320 (manufactured by Beckman Coulter Inc.)", the cumulative distribution of the particle diameter is created from the small diameter side in terms of the volume basis, and the particle diameter that gives 50% of accumulation is determined as the volume average particle diameter.

### <Manufacturing Method for Cellulose Particle>

A manufacturing method for the cellulose particle according to the present exemplary embodiment is, for example, as follows.

### - Manufacturing Step for Cellulose Particle (Mother Particle) -

(1) First, cellulose acylate is dissolved in a poorly water-soluble organic solvent A to prepare a cellulose acylate solution A.
(2) Next, the cellulose acylate solution A is added to a calcium carbonate dispersion liquid obtained by dispersing calcium carbonate in water and stirred to prepare a cellulose acylate solution B.
(3) Next, a mixed solution of carboxymethyl cellulose and water is added to the cellulose acylate solution B and stirred at high speed to prepare a cellulose acylate solution C.
(4) Next, after sodium hydroxide is added to the cellulose acylate solution C, the cellulose acylate dispersion liquid C is heated to remove the poorly water-soluble organic solvent A, and hydrochloric acid is added thereto to form cellulose acylate particles. Then, the cellulose acylate particles are filtered out, and the filtered cellulose acylate particles are dispersed in water to prepare a cellulose acylate particle suspension.
(5) Next, after sodium hydroxide is added to the cellulose acylate particle suspension, the cellulose acylate particle suspension is heated in a weak alkaline environment and stirred to saponificate the cellulose acylate particles to prepare a cellulose particle suspension.
(6) Next, hydrochloric acid is added to the cellulose particle suspension to bring the pH of the suspension to near neutral (for example, in a range of 6.5 or more and 7 or less), and then the cellulose particles are repeatedly subjected to filtering out and washing with pure water. Then, after the conductivity of the filtrate reaches 10 µs/cm or less, the filtered-out cellulose particles are dried.

Here, the cellulose acylate is a cellulose derivative in which at least one of hydroxy groups in cellulose is substituted (acylated) with an aliphatic acyl group. Specifically, the cellulose acylate is a cellulose derivative in which at least one of hydroxy groups in cellulose is substituted with -CO-R^{AC} (R^{AC} represents an aliphatic hydrocarbon group).

The poorly water-soluble organic solvent A is a solvent that dissolves 0.1% by mass or more and 10% by mass of water with respect to the solvent at 25°C, and examples thereof include ethyl acetate, butyl acetate, and methyl ethyl ketone.

### - Coating Layer Forming Step -

First, the mother particles are added to an aqueous solution containing a salt of a basic polysaccharide (for example, a lactic acid salt of chitosan) and stirred.

Next, an alkaline aqueous solution (for example, an aqueous sodium hydroxide solution) is added to this suspension to precipitate the basic polysaccharide on the surface of the mother particle. As a result, the first coating layer containing a basic polysaccharide is formed on the surface of the mother particle.

Here, in a case where an alkaline aqueous solution is not added (that is, in a case where the basic polysaccharide is not precipitated on the surface of the mother particle), the suspension is dried, thereby obtaining the mother particle on which the first coating layer made from a salt of the basic polysaccharide has been formed.

The amount of surface coating by the first coating layer is measured as follows. It is determined as a difference between an amount of the salt of the basic polysaccharide contained in the aqueous solution before the addition of the mother particle and an amount of the basic polysaccharide or salt of the basic polysaccharide, which is obtained by drying and solidifying a supernatant in a case where a suspension containing the mother particles and the basic polysaccharide or salt of the basic polysaccharide (that is, a suspension in a case where an alkaline aqueous solution is added or in a case where the alkaline aqueous solution is not added) is sufficiently stirred and then allowed to stand.

Next, after heating the suspension of the mother particles on which the first coating layer has been formed, a separately heated aqueous solution containing a sodium salt of a hydrophobic compound is added thereto.

Next, a metal chloride or an acid aqueous solution is added to this suspension to precipitate the hydrophobic compound. As a result, the second coating layer made from the hydrophobic compound is formed on the first coating layer.

Here, in a case where a metal chloride is applied, a metal salt of the hydrophobic compound (that is, a fatty acid metal salt or an acylated amino acid metal salt) is precipitated. On the other hand, in a case where an acid aqueous solution is applied, a hydrophobic compound of a non-metal salt (that is, a fatty acid or an acylated amino acid) is precipitated.

Next, cellulose particles having the coating layer are extracted from the suspension. The extraction of the cellulose particles having the coating layer is carried out, for example, by filtering the suspension. The extracted cellulose particles having the coating layer may be washed, for example, with water. As a result, the coating layer forming material which has not been subjected to coating can be removed. Then, the cellulose particles having the coating layer are dried to obtain the cellulose particles according to the present exemplary embodiment.

It is noted that the amount of surface coating by the second coating layer is determined as a difference between an amount of a hydrophobic compound added to the suspension of the mother particles on which the first coating layer has been formed, and an amount of a hydrophobic compound obtained by drying and solidifying a filtrate at the time of extracting the particles in which the second coating layer has been formed.

### - External Addition Step -

An external additive may be added to the obtained cellulose particles.

Examples of the external addition step include a treatment of adding an external additive to the cellulose particles by using a mixing mill, a V-type blender, a Henschel mixer, a Loedige mixer, or the like.

### <Use Application>

Examples of the use application of the cellulose particle according to the present exemplary embodiment include cosmetics, rolling materials, abrasives, scrubbing agents, display spacers, beads molding materials, light diffusing particles, resin strengthening agents, refractive index control agents, biodegradation accelerators, fertilizers, water-absorbent particles, toner particles, and granules of anti-blocking particle.

The use application of the cellulose particle according to the present exemplary embodiment is, for example, preferably cosmetics.

Among the above, the use application of the cellulose particle according to the present exemplary embodiment is, for example, preferably an additive for cosmetics.

The cellulose particle according to the present exemplary embodiment has excellent flexibility, and thus in a case where the cellulose particle is used as an additive for cosmetics, the spread of a cosmetic on the skin tends to be favorable in a case where the cosmetic is applied to the skin.

The cellulose particle according to the present exemplary embodiment can be applied as an additive for cosmetics such as base makeup cosmetics (for example, a makeup base, a concealer, a foundation, and a face powder); makeup cosmetics (for example, a lipstick, a gloss, a lip liner, a blush, an eye shadow, an eyeliner, a mascara, an eyebrow, a nail, and a nail care cosmetic); and skin care cosmetics (for example, a facial wash material, a cleansing, a lotion, an emulsion, a liquid cosmetic, a pack, a face mask, and an eye and mouth care cosmetic).

In particular, from the viewpoint that the cosmetic additive for makeup cosmetics is required to have flexibility and biodegradability, the cellulose particle according to the present exemplary embodiment is used, for example, preferably as a cosmetic additive for makeup cosmetics.

### [Examples]

Hereinafter, Examples will be described below; It is noted that in the following description, all "parts" and "%" are in terms of mass unless otherwise specified.

### <Examples 1 to 33 and 36 to 41, and Comparative Examples 1 to 3>

### - Formation of Mother Particle -

As a cellulose acylate, 130 parts of DAC "L-50" manufactured by Daicel Corporation, cellulose diacetate, weight average polymerization degree: 570" is completely dissolved in 1,070 parts of ethyl acetate. This was added to an aqueous liquid containing 45 parts of calcium carbonate and 500 parts of pure water, and the resultant mixture was stirred for 3 hours. A solution obtained by dispersing 4 parts of carboxymethyl cellulose (hereinafter, also referred to as "CMC") in 600 parts of pure water was added thereto, and the resultant mixture was stirred with a high-speed emulsifier for 5 minutes. 10 parts of sodium hydroxide was added thereto, and the resultant mixture was heated to 80°C and stirred for 3 hours to remove ethyl acetate. The same amount of dilute hydrochloric acid added as the amount of sodium hydroxide was added thereto, and the residue was filtered and then dispersed in pure water again to obtain a suspension of cellulose acylate particles (solid content concentration: 10%).

17.5 parts of a 20% aqueous sodium hydroxide solution was added to 500 parts of the suspension of cellulose acylate particles, and the resultant mixture was stirred for 6 hours at a saponification temperature of 30°C. After adjusting the pH to 7 by adding hydrochloric acid to the suspension after saponification, filtration and washing were repeated, and washing was carried out until the conductivity of the filtrate reached 10 µs/cm or less, thereby obtaining cellulose mother particles.

### (Surface Treatment)

The mother particles were subjected to the following surface treatment.

A cake-shaped filtered-out product obtained by repeating filtration and washing until the conductivity of the filtrate reached 10 µs/cm or less was dried to obtain cellulose mother particles.

Next, the mother particles were added to an aqueous solution containing a lactic acid salt of chitosan, and stirring was at 30°C for 3 hours. Then, an aqueous NaOH solution was added to the aqueous solution to precipitate chitosan on the surface of the mother particle. As a result, the first coating layer having a coating amount shown in Table 1 was formed on the surface of the mother particle.

Next, after heating to 80°C the suspension of the mother particles on which the first coating layer has been formed, and then an aqueous solution separately heated to 80°C, which contained a sodium salt of a fatty acid or acylated amino acid of the kind shown in Table 1, was added, and the resultant mixture was stirred for 6 hours. Then, an aqueous solution of calcium chloride, magnesium chloride, aluminum chloride, zinc chloride or hydrochloric acid was added thereto to form the second coating layer of the kind and coating amount shown in Table 1, on the surface of the mother particle having the first coating layer.

### (Washing Treatment)

Next, a mother particle slurry on which the first and second coating layers were formed was filtered, and the filtered-out product was washed with pure water and then filtered again. This operation was repeated, and in a case where the conductivity of the filtrate reached 10 µs/cm or less, the filtered-out product was freeze-dried to obtain cellulose particles.

It is noted that in a part of the examples (Comparative Examples 1 to 3), cellulose particles in which one or both of the first coating layer and the second coating layer were not formed on the mother particles were prepared.

In addition, in a part of the examples (Examples 32 and 33), chitosan is not precipitated on the surface of the mother particle since the aqueous NaOH solution is not added. That is, a solution obtained by adding the mother particles to an aqueous solution containing a lactic acid salt of chitosan or a hydrochloric acid salt of chitosan was dried to form, on the mother particles, the first coating layer containing the lactic acid salt of chitosan or the hydrochloric acid salt of chitosan. Then, the second coating layer was formed on the mother particles having the first coating layer to prepare cellulose particles.

### <Examples 34 and 35>

As the salt of the basic polysaccharide, sodium hyaluronate was used instead of the lactic acid salt of chitosan.

In Example 34, dilute hydrochloric acid was added instead of the aqueous NaOH solution to the suspension of the mother particles treated with sodium hyaluronate as conditions for the conversion of sodium hyaluronate to hyaluronic acid and the formation of the first coating layer containing hyaluronic acid, whereby the first coating layer was formed. Other operations are the same as in <Examples 1 to 31 and 36 to 41>.

Further, in Example 35, the first coating layer was formed in the same manner as in <Examples 32 and 33>, using the conditions for forming the first coating layer containing sodium hyaluronate. Other operations are the same as in <Examples 1 to 31 and 36 to 41>.

### <Comparative Example 4>

Pullulan was used as the polysaccharide having no primary amine or secondary amine instead of the salt of the basic polysaccharide. The first coating layer and the second coating layer were formed in the same manner as in <Examples 32, 33, 35> regarding the operation.

### <Evaluation of Physical Properties>

### (Volume Average Particle Diameter)

The volume average particle diameter (denoted as "D50v" in the table) of the cellulose particles obtained in each example was measured.

Here, a particle diameter is measured by an LS particle diameter distribution measuring apparatus "Beckman Coulter LS13 320 (manufactured by Beckman Coulter Inc.)", the cumulative distribution of the particle diameter is created from the small diameter side in terms of the volume basis, and the particle diameter that gives 50% of accumulation is determined as the volume average particle diameter.

### <Evaluation>

### (Water Repellency)

50 mL of ion exchange water was added to a 100 mL sample bottle, and then 5 g of the cellulose particles of each example were charged thereinto. Then, the sample bottle was immersed in a hot water bath at 65°C for one week. After storage for one week, the completely floating cellulose particles were collected, the weight thereof after drying was measured, and the water repellency of the cellulose particles was evaluated according to the evaluation standards shown below. The cellulose particles before storage were also subjected the same evaluation. It is noted that the water repellency is determined to be excellent in a case where the water repellency evaluation after storage is A to E, and the water repellency is determined to be inferior in a case where the water repellency evaluation after storage is F. A has the highest water repellency, and F has the lowest water repellency.
A: The weight of the completely floating cellulose particles is more than 90% and 100% or less with respect to the weight of the particles initially charged.
B: The weight of the completely floating cellulose particles is more than 80% and 90% or less with respect to the weight of the particles initially charged.
C: The weight of the completely floating cellulose particles is more than 70% and 80% or less with respect to the weight of the particles initially charged.
D: The weight of the completely floating cellulose particles is more than 60% and 70% or less with respect to the weight of the particles initially charged.
E: The weight of the completely floating cellulose particles is more than 50% and 60% or less with respect to the weight of the particles initially charged.
F: The weight of the completely floating cellulose particles is 50% or less with respect to the weight of the particles initially charged.

### (Moist Feeling)

The cellulose particles of each of examples before and after the storage in the hot water bath at 65°C were subjected, by twenty panelists, to five-grade evaluations of grades 1 to 5 on the "moist feeling" in a case of being applied onto the skin. The graded 5 has the strongest moist feeling. According to the evaluation standards shown below, the average value of the five-grade evaluations of twenty people was re-denoted as A to F, and then the moist feeling was determined to be excellent in a case where the evaluation after storage is A to E, and the moist feeling was determined to be inferior in a case where the evaluation after storage is F. A has the strongest moist feeling, and F has the weakest moist feeling.

It is noted that a coating sample after storage in a hot water bath at 65°C was prepared as follows.

50 mL of ion exchange water was added to a 100 mL sample bottle, and then 5 g of the cellulose particles of each example were charged thereinto. Then, the sample bottle was immersed in a hot water bath at 65°C for one week. After storage for one week, the cellulose particles were collected and dried to prepare a coating sample.
A: The average value of moist feeling is 4.5 or more.
B: The average value of moist feeling is 4 or more and less than 4.5.
C: The average value of moist feeling is 3.5 or more and less than 4.
D: The average value of moist feeling is 3 or more and less than 3.5.
E: The average value of moist feeling is 2.5 or more and less than 3.
F: The average value of moist feeling is less than 2.5.

**[Table 1-1]**

| | Mother particle | First coating layer | | Second coating layer | | Coating amount ratio A/B | D50v (µm) |
|---|---|---|---|---|---|---|---|
| | Resin kind before saponification | Kind | Coating amount A (%) (with respect to mother particle) | Kind | Coating amount B (%) (with respect to mother particle) | | |
| Example 1 | DAC | Chitosan | 1 | Calcium stearate | 5 | 0.20 | 6 |
| Example 2 | DAC | Chitosan | 0.25 | Calcium stearate | 5 | 0.05 | 6 |
| Example 3 | DAC | Chitosan | 3 | Calcium stearate | 5 | 0.60 | 6 |
| Example 4 | DAC | Chitosan | 0.2 | Calcium stearate | 5 | 0.04 | 6 |
| Example 5 | DAC | Chitosan | 10 | Calcium stearate | 5 | 2.00 | 6 |
| Example 6 | DAC | Chitosan | 1 | Calcium stearate | 3 | 0.33 | 6 |
| Example 7 | DAC | Chitosan | 1 | Calcium stearate | 12 | 0.08 | 6 |
| Example 8 | DAC | Chitosan | 1 | Calcium stearate | 0.5 | 2.00 | 6 |
| Example 9 | DAC | Chitosan | 1 | Calcium stearate | 25 | 0.04 | 6 |
| Example 10 | DAC | Chitosan | 1 | Calcium stearate | 5 | 0.20 | 3 |
| Example 11 | DAC | Chitosan | 1 | Calcium stearate | 5 | 0.20 | 10 |
| Example 12 | DAC | Chitosan | 1 | Magnesium stearate | 5 | 0.20 | 6 |
| Example 13 | DAC | Chitosan | 1 | Aluminum stearate | 5 | 0.20 | 6 |
| Example 14 | DAC | Chitosan | 1 | Zinc stearate | 5 | 0.20 | 6 |
| Example 15 | DAC | Chitosan | 1 | Calcium myristate | 5 | 0.20 | 6 |
| Example 16 | DAC | Chitosan | 1 | Magnesium myristate | 5 | 0.20 | 6 |
| Example 17 | DAC | Chitosan | 1 | Aluminum myristate | 5 | 0.20 | 6 |
| Example 18 | DAC | Chitosan | 1 | Zinc myristate | 5 | 0.20 | 6 |
| Example 19 | DAC | Chitosan | 1 | Calcium behenate | 5 | 0.20 | 6 |
| Example 20 | DAC | Chitosan | 1 | Zinc palmitate | 5 | 0.20 | 6 |
| Example 21 | DAC | Chitosan | 1 | Aluminum myristoyl glutamate | 5 | 0.20 | 6 |
| Example 22 | DAC | Chitosan | 1 | Aluminum stearoyl glutamate | 5 | 0.20 | 6 |
| Example 23 | DAC | Chitosan | 1 | Magnesium palmitic glutamate | 5 | 0.20 | 6 |
| Example 24 | DAC | Chitosan | 1 | Calcium myristoyl glutamate | 5 | 0.20 | 6 |
| Example 25 | DAC | Chitosan | 1 | Stearic acid | 5 | 0.20 | 6 |
| Example 26 | DAC | Chitosan | 1 | Behenic acid | 5 | 0.20 | 6 |
| Example 27 | DAC | Chitosan | 1 | Lauroyl lysine | 5 | 0.20 | 6 |
| Example 28 | DAC | Chitosan | 1 | Myristoyl glutamate | 5 | 0.20 | 6 |
| Example 29 | DAC | Chitosan | 1 | Lauroyl glutamate | 5 | 0.20 | 6 |
| Example 30 | DAC | Chitosan | 1 | Lauroyl aspartate | 5 | 0.20 | 6 |
| Example 31 | DAC | Chitosan | 1 | Stearoyl glutamate | 5 | 0.20 | 6 |
| Example 32 | DAC | Lactic acid salt of chitosan | 1 | Calcium stearate | 5 | 0.20 | 6 |
| Example 33 | DAC | Hydrochloric acid salt of chitosan | 1 | Calcium stearate | 5 | 0.20 | 6 |
| Example 34 | DAC | Hyaluronic acid | 1 | Calcium stearate | 5 | 0.20 | 6 |
| Example 35 | DAC | Hyaluronic acid sodium salt | 1 | Calcium stearate | 5 | 0.20 | 6 |
| Example 36 | DAC | Chitosan | 0.05 | Calcium stearate | 5 | 0.01 | 6 |
| Example 37 | DAC | Chitosan | 15 | Calcium stearate | 5 | 3.00 | 6 |
| Example 38 | DAC | Chitosan | 1 | Calcium stearate | 0.3 | 3.33 | 6 |
| Example 39 | DAC | Chitosan | 1 | Calcium stearate | 30 | 0.03 | 6 |
| Example 40 | DAC | Chitosan | 1 | Calcium stearate | 5 | 0.20 | 2 |
| Example 41 | DAC | Chitosan | 1 | Calcium stearate | 5 | 0.20 | 15 |
| Comparative Example 1 | DAC | Chitosan | 1 | - | 0 | - | 6 |
| Comparative Example 2 | DAC | - | 0 | Calcium stearate | 1 | - | 6 |
| Comparative Example 3 | DAC | - | 0 | - | 0 | - | 6 |
| Comparative Example 4 | DAC | Pullulan | 1 | Calcium stearate | 5 | 0.20 | 6 |

**[Table 1-2]**

| | Evaluation of water repellency before storage | Evaluation of water repellency after storage | Average value of moist feeling before storage according to evaluation by panelist | Average value of moist feeling after storage according to evaluation by panelist | Evaluation of moist feeling after storage |
|---|---|---|---|---|---|
| Example 1 | A | A | 4.6 | 4.6 | A |
| Example 2 | A | B | 4.2 | 4.1 | B |
| Example 3 | A | B | 4.3 | 4.3 | B |
| Example 4 | B | C | 4.0 | 3.8 | C |
| Example 5 | B | C | 4.1 | 3.9 | C |
| Example 6 | A | B | 4.2 | 4.0 | B |
| Example 7 | A | B | 4.3 | 4.1 | B |
| Example 8 | B | C | 3.9 | 3.6 | C |
| Example 9 | B | C | 4.2 | 3.7 | C |
| Example 10 | B | C | 4.0 | 3.6 | C |
| Example 11 | B | C | 4.2 | 3.8 | C |
| Example 12 | A | B | 4.2 | 4.1 | B |
| Example 13 | A | B | 4.1 | 4.0 | B |
| Example 14 | B | C | 4.1 | 4.0 | B |
| Example 15 | B | C | 4.3 | 4.1 | B |
| Example 16 | B | C | 4.2 | 4.0 | B |
| Example 17 | B | C | 4.2 | 4.1 | B |
| Example 18 | B | C | 4.3 | 4.0 | B |
| Example 19 | B | C | 4.0 | 4.0 | B |
| Example 20 | B | C | 4.2 | 4.0 | B |
| Example 21 | B | C | 3.9 | 3.7 | C |
| Example 22 | B | C | 4.2 | 3.8 | C |
| Example 23 | C | D | 3.9 | 3.1 | D |
| Example 24 | B | D | 3.8 | 3.3 | D |
| Example 25 | C | E | 3.6 | 2.8 | E |
| Example 26 | C | E | 3.7 | 2.8 | E |
| Example 27 | C | E | 3.6 | 2.6 | E |
| Example 28 | C | E | 3.5 | 2.7 | E |
| Example 29 | C | E | 3.5 | 2.6 | E |
| Example 30 | C | E | 3.5 | 2.6 | E |
| Example 31 | C | E | 3.6 | 2.8 | E |
| Example 32 | C | D | 3.6 | 3.2 | D |
| Example 33 | D | E | 3.2 | 2.7 | E |
| Example 34 | D | E | 3.0 | 2.6 | E |
| Example 35 | D | E | 3.0 | 2.5 | E |
| Example 36 | D | E | 3.1 | 2.5 | E |
| Example 37 | D | E | 3.2 | 2.6 | E |
| Example 38 | D | E | 3.1 | 2.6 | E |
| Example 39 | D | E | 3.2 | 2.7 | E |
| Example 40 | D | E | 3.2 | 2.5 | E |
| Example 41 | D | E | 3.3 | 2.7 | E |
| Comparative Example 1 | F | F | 2.7 | 1.9 | F |
| Comparative Example 2 | F | F | 26 | 1.7 | F |
| Comparative Example 3 | F | F | 1.5 | 1.5 | F |
| Comparative Example 4 | F | F | 2.5 | 1.9 | F |

From the above results, it can be seen that the cellulose particles of the present Example are excellent in water repellency and moist feeling even in a case of being exposed to high temperature water for a long period of time as compared with the cellulose particles of Comparative Examples.

The effects of the above-described aspects are as follows.

There is provided a cellulose particle excellent in water repellency and moist feeling even in a case of being exposed to high temperature water for a long period of time, as compared with a cellulose particle having a mother particle containing cellulose as a main component, where the cellulose particle has, in the following order, a first coating layer that contains a cellulose particle having a single coating layer on the surface of the mother particle or contains a polysaccharide having no primary amine or secondary amine on the surface of the mother particle, and a second coating layer containing calcium stearate.

There is provided a cellulose particle excellent in water repellency and moist feeling even in a case of being exposed to high temperature water for a long period of time, as compared with a case where the polysaccharide is a lactic acid salt of chitosan.

There is provided a cellulose particle excellent in water repellency and moist feeling even in a case of being exposed to high temperature water for a long period of time, as compared with a case where the hydrophobic compound is at least one selected from a fatty acid or an acylated amino acid.

There is provided a cellulose particle excellent in water repellency and moist feeling even in a case of being exposed to high temperature water for a long period of time, as compared with a case where the hydrophobic compound is an acylated amino acid salt.

There is provided a cellulose particle excellent in water repellency and moist feeling even in a case of being exposed to high temperature water for a long period of time, as compared with a case where the fatty acid metal salt is magnesium stearate.

There is provided a cellulose particle excellent in water repellency and moist feeling even in a case of being exposed to high temperature water for a long period of time, as compared with a case where the acylated amino acid salt is calcium myristoyl glutamate.

There is provided a cellulose particle excellent in water repellency and moist feeling even in a case of being exposed to high temperature water for a long period of time, as compared with a case where the polysaccharide is a lactic acid salt of chitosan or the hydrophobic compound is magnesium stearate.

There is provided a cellulose particle in which the coating amount of the first coating layer with respect to the mother particle is less than 0.2% by mass or more than 10% by mass.

There is provided a cellulose particle excellent in water repellency and moist feeling even in a case of being exposed to high temperature water for a long period of time, as compared with a case where the coating amount of the first coating layer with respect to the mother particle is less than 0.25% by mass or more than 3% by mass.

There is provided a cellulose particle excellent in water repellency and moist feeling even in a case of being exposed to high temperature water for a long period of time, as compared with a case where the coating amount of the second coating layer with respect to the mother particle is less than 0.5% by mass or more than 25% by mass.

There is provided a cellulose particle excellent in water repellency and moist feeling even in a case of being exposed to high temperature water for a long period of time, as compared with a case where the coating amount of the second coating layer with respect to the mother particle is less than 3% by mass or more than 12% by mass.

There is provided a cellulose particle excellent in water repellency and moist feeling even in a case of being exposed to high temperature water for a long period of time, as compared with a case where the ratio of the first coating amount to the coating amount of the second coating layer is less than 0.04 or more than 2.

There is provided a cellulose particle excellent in water repellency and moist feeling even in a case of being exposed to high temperature water for a long period of time, as compared with a case where the volume average particle diameter of the cellulose particles is less than 3 µm or more than 10 µm.

The foregoing description of the exemplary embodiments of the present invention has been provided for the purposes of illustration and description.

## Claims

1. A cellulose particle comprising:
a mother particle containing cellulose as a main component;
a first coating layer containing at least one polysaccharide selected from the group consisting of a basic polysaccharide and a salt of the basic polysaccharide, the first coating layer being provided on a surface of the mother particle; and
a second coating layer containing at least one hydrophobic compound selected from a fatty acid, a fatty acid metal salt, an acylated amino acid, or an acylated amino acid salt, the second coating layer being provided on the first coating layer.

2. The cellulose particle according to claim 1,
wherein the polysaccharide is chitosan.

3. The cellulose particle according to claim 1 or 2,
wherein the hydrophobic compound is at least one selected from a fatty acid metal salt or an acylated amino acid salt.

4. The cellulose particle according to claim 3,
wherein the hydrophobic compound is a fatty acid metal salt.

5. The cellulose particle according to claim 3 or 4,
wherein the fatty acid metal salt is calcium stearate.

6. The cellulose particle according to any one of claims 3 to 5,
wherein the acylated amino acid salt is at least one selected from aluminum stearoyl glutamate or aluminum myristoyl glutamate.

7. The cellulose particle according to any one of claims 1 to 6,
wherein the polysaccharide is chitosan, and
the hydrophobic compound is calcium stearate.

8. The cellulose particle according to any one of claims 1 to 7,
wherein a coating amount of the first coating layer with respect to the mother particle is 0.2% by mass or more and 10% by mass or less.

9. The cellulose particle according to claim 8,
wherein the coating amount of the first coating layer with respect to the mother particle is 0.25% by mass or more and 3% by mass or less.

10. The cellulose particle according to any one of claims 1 to 9,
wherein a coating amount of the second coating layer with respect to the mother particle is 0.5% by mass or more and 25% by mass or less.

11. The cellulose particle according to claim 10,
wherein the coating amount of the second coating layer with respect to the mother particle is 3% by mass or more and 12% by mass or less.

12. The cellulose particle according to any one of claims 1 to 11,
wherein a ratio of a coating amount of the first coating layer to a coating amount of the second coating layer is 0.04 or more and 2 or less.

13. The cellulose particle according to any one of claims 1 to 12,
wherein a volume average particle diameter of the cellulose particles is 3 µm or more and 10 µm or less as determined by the methods disclosed in the description.

## Patentansprüche

1. Zellulosepartikel, umfassend:
ein Mutterpartikel, das Zellulose als eine Hauptkomponente enthält;
eine erste Beschichtungsschicht, die mindestens ein Polysaccharid, das aus der Gruppe, die aus einem basischen Polysaccharid und einem Salz des basischen Polysaccharids besteht, ausgewählt wird, enthält, wobei die erste Beschichtungsschicht auf einer Oberfläche des Mutterpartikels vorgesehen ist; und
eine zweite Beschichtungsschicht, die mindestens eine hydrophobe Verbindung, die aus einer Fettsäure, einem Fettsäure-Metallsalz, einer acylierten Aminosäure und einem acylierten Aminosäuresalz ausgewählt wird, enthält, wobei die zweite Beschichtungsschicht auf der ersten Beschichtungsschicht vorgesehen ist.

2. Zellulosepartikel nach Anspruch 1,
wobei das Polysaccharid Chitosan ist.

3. Zellulosepartikel nach Anspruch 1 oder 2,
wobei die hydrophobe Verbindung mindestens eines ist, das aus einem Fettsäure-Metallsalz und einem acylierten Aminosäuresalz ausgewählt wird.

4. Zellulosepartikel nach Anspruch 3,
wobei die hydrophobe Verbindung ein Fettsäure-Metallsalz ist.

5. Zellulosepartikel nach Anspruch 3 oder 4,
wobei das Fettsäure-Metallsalz Calciumstearat ist.

6. Zellulosepartikel nach einem der Ansprüche 3 bis 5,
wobei das acylierte Aminosäuresalz mindestens eines, das aus Aluminiumstearoylglutamat und Aluminiummyristoylglutamat ausgewählt wird, ist.

7. Zellulosepartikel nach einem der Ansprüche 1 bis 6,
wobei das Polysaccharid Chitosan ist, und
die hydrophobe Verbindung Calciumstearat ist.

8. Zellulosepartikel nach einem der Ansprüche 1 bis 7,
wobei eine Beschichtungsmenge der ersten Beschichtungsschicht in Bezug auf das Mutterpartikel 0,2 Massen-% oder mehr und 10 Massen-% oder weniger beträgt.

9. Zellulosepartikel nach Anspruch 8,
wobei die Beschichtungsmenge der ersten Beschichtungsschicht in Bezug auf das Mutterpartikel 0,25 Massen-% oder mehr und 3 Massen-% oder weniger beträgt.

10. Zellulosepartikel nach einem der Ansprüche 1 bis 9,
wobei eine Beschichtungsmenge der zweiten Beschichtungsschicht in Bezug auf das Mutterpartikel 0,5 Massen-% oder mehr und 25 Massen-% oder weniger beträgt.

11. Zellulosepartikel nach Anspruch 10,
wobei die Beschichtungsmenge der zweiten Beschichtungsschicht in Bezug auf das Mutterpartikel 3 Massen-% oder mehr und 12 Massen-% oder weniger beträgt.

12. Zellulosepartikel nach einem der Ansprüche 1 bis 11,
wobei ein Verhältnis einer Beschichtungsmenge der ersten Beschichtungsschicht zu einer Beschichtungsmenge der zweiten Beschichtungsschicht 0,04 oder mehr und 2 oder weniger beträgt.

13. Zellulosepartikel nach einem der Ansprüche 1 bis 12,
wobei ein volumendurchschnittliche Partikeldurchmesser der Zellulosepartikel 3 µm oder mehr und 10 µm oder weniger, wie durch die in der Beschreibung offenbarten Verfahren bestimmt, beträgt.

## Revendications

1. Particule de cellulose comprenant :
une particule mère contenant de la cellulose comme composant principal ;
une première couche de revêtement contenant au moins un polysaccharide choisi dans le groupe constitué d'un polysaccharide basique et d'un sel du polysaccharide basique, la première couche de revêtement étant disposée sur une surface de la particule mère ; et
une deuxième couche de revêtement contenant au moins un composé hydrophobe choisi parmi un acide gras, un sel de métal d'acide gras, un acide aminé acylé ou un sel d'acide aminé acylé, la deuxième couche de revêtement étant disposée sur la première couche de revêtement.

2. Particule de cellulose selon la revendication 1,
dans laquelle le polysaccharide est le chitosane.

3. Particule de cellulose selon la revendication 1 ou la revendication 2,
dans laquelle le composé hydrophobe est au moins l'un choisi parmi un sel de métal d'acide gras ou un sel d'acide aminé acylé.

4. Particule de cellulose selon la revendication 3,
dans laquelle le composé hydrophobe est un sel de métal d'acide gras.

5. Particule de cellulose selon la revendication 3 ou la revendication 4,
dans laquelle le sel de métal d'acide gras est le stéarate de calcium.

6. Particule de cellulose selon l'une quelconque des revendications 3 à 5,
dans laquelle le sel d'acide aminé acylé est au moins l'un choisi parmi le stéaroylglutamate d'aluminium ou le myristoylglutamate d'aluminium.

7. Particule de cellulose selon l'une quelconque des revendications 1 à 6,
dans laquelle le polysaccharide est le chitosane, et
le composé hydrophobe est le stéarate de calcium.

8. Particule de cellulose selon l'une quelconque des revendications 1 à 7,
dans laquelle une quantité de revêtement de la première couche de revêtement par rapport à la particule mère est de 0,2 % en masse ou plus et de 10 % en masse ou moins.

9. Particule de cellulose selon la revendication 8,
dans laquelle la quantité de revêtement de la première couche de revêtement par rapport à la particule mère est de 0,25 % en masse ou plus et de 3 % en masse ou moins.

10. Particule de cellulose selon l'une quelconque des revendications 1 à 9,
dans laquelle une quantité de revêtement de la deuxième couche de revêtement par rapport à la particule mère est de 0,5 % en masse ou plus et de 25 % en masse ou moins.

11. Particule de cellulose selon la revendication 10,
dans laquelle la quantité de revêtement de la deuxième couche de revêtement par rapport à la particule mère est de 3 % en masse ou plus et de 12 % en masse ou moins.

12. Particule de cellulose selon l'une quelconque des revendications 1 à 11,
dans laquelle un rapport entre une quantité de revêtement de la première couche de revêtement et une quantité de revêtement de la deuxième couche de revêtement est de 0,04 ou plus et de 2 ou moins.

13. Particule de cellulose selon l'une quelconque des revendications 1 à 12,
dans laquelle un diamètre moyen en volume des particules de cellulose est de 3 µm ou plus et de 10 µm ou moins tel que déterminé par les méthodes divulguées dans la description.
